# EUROPEAN PATENT APPLICATION

(11) **EP 4 534 142 A1**
(43) Date of publication of application: **09.04.2025**
(21) Application number: 23731079.2
(22) Date of filing: 22.03.2023
(51) Int. Cl.: A61N 7/00, A61B 18/26

(54) **EXTRACORPOREAL SHOCK WAVE GENERATION DEVICE**

(30) Priority: 02.06.2022 KR 20220067771
(71) Applicant: Jskbiomed Inc., Daejeon 34028 (KR)
(72) Inventor: JEON, Jinwoo, Sejong 30101 (KR)
(74) Representative: Frenkel, Matthias Alexander
(86) International application number: PCT/KR2023/003818
(87) International publication number: WO 2023/234527

(57) **Abstract**

Provided is an extracorporeal shock wave generator which comprises a housing in which an inner space is filled with a shock wave generating medium, a window coupled to the housing to transmit a laser beam from an outside of the housing to an inside of the housing and a shock wave transmission unit coupled to the housing and configured to transmit a shock wave generated as the laser beam reacts to the shock wave generating medium inside the housing to the outside.

## Description

### TECHNICAL FIELD

The present invention relates to an extracorporeal shock wave generator, and more particularly, to an extracorporeal shock wave generator having a structure capable of effectively transmitting shock waves generated by reacting a laser beam to a shock wave generating medium filled in a housing to an object through a shock wave transmission unit.

### BACKGROUND ART

Extracorporeal shock wave therapy is a treatment method of relieving pain by directly applying shock waves to a painful part of the body, and may be used to treat various diseases such as shoulder pain (calcified tendinitis, rotator cuff tendinitis, frozen shoulder), elbow pain, knee pain (degenerative arthritis, pes anserinus tendititis), achilles tendinitis, plantar pain (plantar fasciitis), hip pain (greater trochanteric pain syndrome, greater trochanteric bursitis), and muscle pain (fasciitis pain syndrome).

Although extracorporeal shock wave therapy is a non-surgical treatment method, the treatment effect is good, daily life is possible immediately after treatment, and the treatment time required is short, so it can be treated conveniently.

### DESCRIPTION OF EMBODIMENTS

### TECHNICAL PROBLEM

The present invention provides an extracorporeal shock wave generator having a structure capable of effectively transmitting shock waves generated by reacting a laser beam to a shock wave generating medium filled in a housing to an object through a shock wave transmission unit.

### SOLUTION TO PROBLEM

According to an aspect of an embodiment, an extracorporeal shock wave generator may comprise a housing in which an inner space is filled with a shock wave generating medium, a window coupled to the housing to transmit a laser beam from an outside of the housing to an inside of the housing and a shock wave transmission unit coupled to the housing and configured to transmit a shock wave generated as the laser beam reacts to the shock wave generating medium inside the housing to the outside.

According to the embodiment, the extracorporeal shock wave generator may further comprise a coupling unit configured to couple the housing and the shock wave transmission unit.

According to the embodiment, the extracorporeal shock wave generator may further comprise a buffer unit configured to reduce a intensity of the generated shock wave.

According to the embodiment, the buffer unit has elasticity and is formed in a plate shape.

According to the embodiment, the buffer unit may be placed in close contact with a cover covering an opening of the housing or one surface of the shock wave transmission unit.

According to the embodiment, the shock wave transmission unit may be an acoustic lens having one surface flat and the other surface concave.

According to the embodiment, when the extracorporeal shock wave generator transmits a shock wave to an object on which a gel-state transmission medium is applied, as the transmission medium flows into a concave portion of the shock wave transmission unit, the shock wave transmission unit may come into close contact with the object.

According to the embodiment, the shock wave transmission unit may be an acoustic lens having one surface flat and the other surface convex.

According to the embodiment, the extracorporeal shock wave generator may further comprise a buffer unit configured to seal a first space filled with the shock wave generating medium in the housing, and a second space sealed by the buffer unit and the shock wave transmission unit is filled with a shock wave transmission medium.

According to the embodiment, the shock wave transmission unit may be formed in a convex cap shape in a direction in which a shock wave is transmitted.

According to the embodiment, the shock wave transmission unit may be formed in a convex cap shape in a direction in which a shock wave is transmitted, and an end thereof may be formed in a flat surface shape.

According to the embodiment, the window may be composed of a lens capable of condensing the laser beam and may be movable in height according to a set target shock wave transmission depth.

According to the embodiment, the extracorporeal shock wave generator may further comprise a shock wave generating medium adjustor configured to control an amount of shock wave generating medium in the first space according to height movement of the window.

According to the embodiment, a top portion of the inner space of the housing may have a dome-shaped cross section.

According to the embodiment, the shock wave transmission unit may be formed of a low-density plastic material.

### ADVANTAGEOUS EFFECTS OF DISCLOSURE

A device according to an embodiment of the present invention may effectively transmit a shock wave generated by reacting a laser beam to a shock wave generating medium filled in a housing to an object through a shock wave transmission unit.

A device according to an embodiment of the present invention may adjust a shock wave generated through a buffer unit provided in the device to an intensity suitable for extracorporeal shock wave therapy.

A device according to an embodiment of the present invention may effectively adjust a shock wave transmission depth transmitted to an object by implementing a shock wave transmission unit provided in the device as a concave or convex acoustic lens.

A device according to an embodiment of the present invention may include a lens capable of condensing a laser beam in the device, and may adjust a set target shock wave transmission depth by moving a height of the lens.

A device according to an embodiment of the present invention may effectively transmit a shock wave generated according to reaction between a shock wave generating medium inside a housing and laser beam to an object by implementing a top portion of an inner space of the housing to have a dome-shaped cross-section.

### BRIEF DESCRIPTION OF DRAWINGS

A brief description of each drawing is provided to more fully understand drawings recited in the detailed description of the present invention.
FIG. 1 is a view of a structure of an extracorporeal shock wave generator according to an embodiment of the present invention.
FIG. 2 is an exploded view of the extracorporeal shock wave generator shown in FIG. 1.
FIG. 3 is a view showing another embodiment of a shock wave transmission unit shown in FIG. 1.
FIG. 4 is a view of a structure of an extracorporeal shock wave generator according to another embodiment of the present invention.
FIG. 5 is an exploded view of the extracorporeal shock wave generator shown in FIG. 4.
FIG. 6 is a view showing another embodiment of a shock wave transmission unit shown in FIG. 4.
FIG. 7 is a view of a structure of an extracorporeal shock wave generator according to another embodiment of the present invention.

### MODE OF DISCLOSURE

Since the disclosure may have diverse modified embodiments, preferred embodiments are illustrated in the drawings and are described in the detailed description. However, this is not intended to limit the disclosure to particular modes of practice, and it is to be appreciated that all changes, equivalents, and substitutes that do not depart from the spirit and technical scope of the disclosure are encompassed in the disclosure.

In the description of the disclosure, certain detailed explanations of the related art are omitted when it is deemed that they may unnecessarily obscure the essence of the disclosure. In addition, numeral figures (e.g., first, second, and the like) used during describing the specification are just identification symbols for distinguishing one element from another element.

Further, in the specification, if it is described that one component is "connected" or "accesses" the other component, it is understood that the one component may be directly connected to or may directly access the other component but unless explicitly described to the contrary, another component may be "connected" or "access" between the components.

In addition, terms including "unit," "er," "or," "module," and the like disclosed in the specification mean a unit that processes at least one function or operation and this may be implemented by hardware or software such as a processor, a micro processor, a micro controller, a central processing unit (CPU), a graphics processing unit (GPU), an accelerated Processing unit (APU), a digital signal processor (DSP), an application specific integrated circuit (ASIC), and a field programmable gate array (FPGA) or a combination of hardware and software. Furthermore, the terms may be implemented in a form coupled to a memory that stores data necessary for processing at least one function or operation.

In addition, it is intended to clarify that the division of the components in the specification is only made for each main function that each component is responsible for. That is, two or more components to be described later below may be combined into one component, or one component may be divided into two or more components according to more subdivided functions. In addition, it goes without saying that each of the components to be described later below may additionally perform some or all of the functions of other components in addition to its own main function, and some of the main functions that each of the components is responsible for may be dedicated and performed by other components.

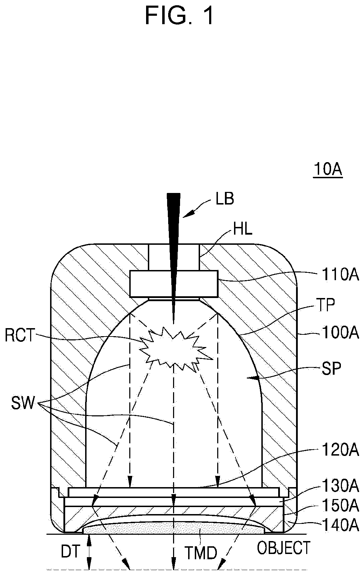

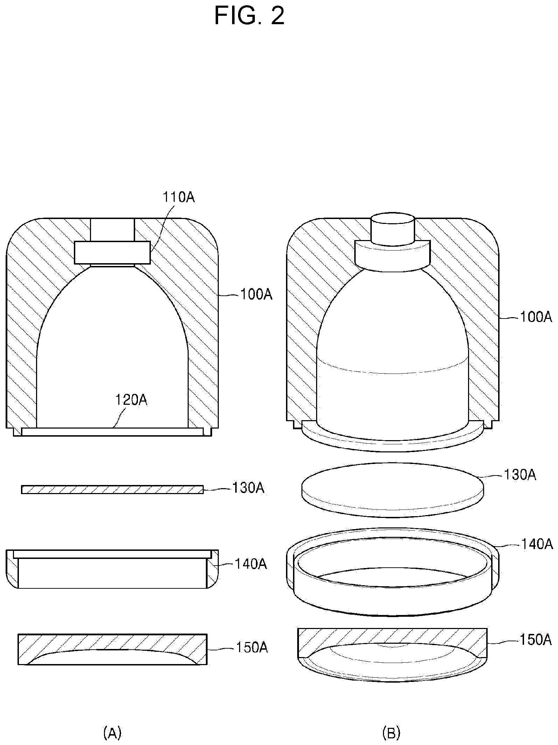

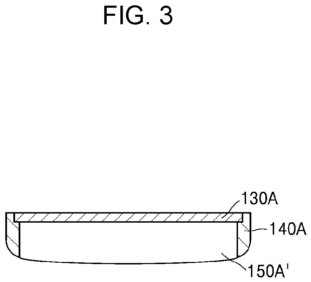

FIG. 1 is a view of a structure of an extracorporeal shock wave generator according to an embodiment of the present invention. FIG. 2 is an exploded view of the extracorporeal shock wave generator shown in FIG. 1. FIG. 3 is a view showing another embodiment of a shock wave transmission unit shown in FIG. 1.

Referring to FIGS. 1 and 2, an extracorporeal shock wave generator 10A according to an embodiment of the present invention may include a housing 100A, a window 110A, a buffer unit 120A, a cover 130A, a coupling unit 140A, and a shock wave transmission unit 150A.

The housing 100A is a part forming the body of the extracorporeal shock wave generator 10A, and a user may hold an outer surface of the housing 100A and use the extracorporeal shock wave generator 10A.

An inner space SP of the housing 100A may be filled with a shock wave generating medium.

According to the embodiment, the shock wave generating medium may correspond to various materials capable of generating a shock wave by reacting with a laser beam LB.

According to an embodiment, the shock wave generating medium may be composed of solid, liquid, gas, an intermediate state thereof, or a combination thereof.

According to an embodiment, the shock wave generating medium may be water or water to which additives are added to adjust the degree of reaction with the laser beam LB.

According to an embodiment, a top portion TP of the inner space SP of the housing 100A may be formed to have a dome-shaped cross section. In this case, among shock waves SW generated by reaction RCT between the laser beam LB and the shock wave generating medium, shock waves having a transmission direction on the opposite side of an object OBJECT are reflected toward the object OBJECT, thereby improving shock wave generation efficiency.

According to an embodiment, when the top portion TP of the inner space SP of the housing 100A is formed to have a dome-shaped cross section, a dome-shaped inclination may be determined according to a location (or height) set to condense the laser beam LB. For example, as a location where the laser beam LB is condensed is relatively close to the object OBJECT, the dome-shaped inclination may be steep, and as a location where the laser beam LB is condensed is relatively distant from the object OBJECT, the dome-shaped inclination may be gentle.

A through hole HL may be formed in an upper portion of the housing 100A to pass the laser beam LB.

The window 110A is provided on the housing 100A and transmits the laser beam LB from the outside of the housing 100A to the inside of the housing 100A.

According to an embodiment, the window 110A may be mounted in a groove formed on the through hole HL of the housing 100A.

According to an embodiment, the window 110A may be formed of a material capable of transmitting the laser beam LB.

According to an embodiment, the window 110A may be formed in a plate-like structure that simply transmits the laser beam LB.

According to another embodiment, the window 110A may be formed of a lens capable of condensing the laser beam LB, for example, a convex lens.

The buffer unit 120A may reduce the intensity of a shock wave SW generated by reaction between the shock wave generating medium and the laser beam LB.

According to an embodiment, the buffer unit 120A may have elasticity and be formed in a plate shape.

According to an embodiment, the buffer unit 120A may be formed of a silicon material.

According to an embodiment, the buffer unit 120A may be placed in close contact with the cover 130A covering an opening of the housing 100A or one surface of the shock wave transmission unit 150A.

According to an embodiment, an open area of the housing 100A is sealed by the buffer unit 120A or the cover 130A, and the sealed inner space SP may be filled with a shock wave generating medium.

According to an embodiment, the extracorporeal shock wave generator 10A may not include the buffer unit 120A or the cover 130A. In this case, the extracorporeal shock wave generator 10A may further include a sealing member (not shown) for sealing the inner space SP of the housing 100A.

The shock wave transmission unit 150A may be coupled to the housing 100A through the coupling unit 140A.

According to an embodiment, the shock wave transmission unit 150A may be directly coupled to the housing 100A without the coupling unit 140A.

The shock wave transmission unit 150A may transmit a shock wave SW generated because the laser beam LB reacts to the shock wave generating medium filled in the inner space SP of the housing 100A to the outside of the housing 100A.

The shock wave transmitted by the shock wave transmission unit 150A may be transmitted to the object OBJECT.

According to an embodiment, the shock wave transmission unit 150A may be formed of low-density plastic, such as low-density polyethylene (LDPE), or linear low-density polyethylene (LLDPE).

Depending on the shape of the shock wave transmission unit 150A, a depth DT at which a shock wave is transmitted to the inside of the object OBJECT may vary.

According to an embodiment, the shock wave transmission unit 150A may be an acoustic lens having one surface flat and the other surface concave. In this case, the shock wave transmission unit 150A may concentrate a shock wave transmitted to the object OBJECT, narrow a range through which the shock wave is transmitted to the object OBJECT, and relatively deepen the depth DT at which the shock wave is transmitted to the inside of the object OBJECT.

For example, when the extracorporeal shock wave generator 10A transmits a shock wave to an object OBJECT on which a gel-state transmission medium TMD is applied, as the transmission medium TMD flows into a concave portion of the shock wave transmission unit 150A, the shock wave transmission unit 150A may come into close contact with the object OBJECT.

Referring to FIG. 3 together, according to an embodiment, a shock wave transmission unit 150A' may be an acoustic lens having one surface flat and the other surface convex. In this case, the shock wave transmission unit 150A' may spread a shock wave transmitted to object OBJECT, thereby widening a range through which the shock wave is transmitted to the object OBJECT, and making the depth DT at which the shock wave is transmitted to the inside of the object OBJECT relatively shallow.

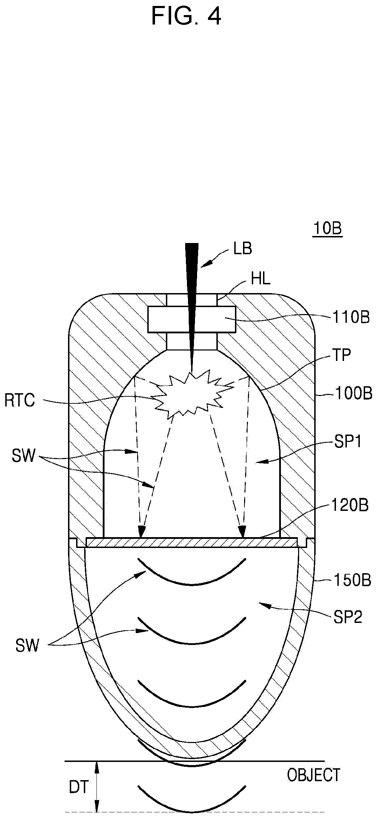

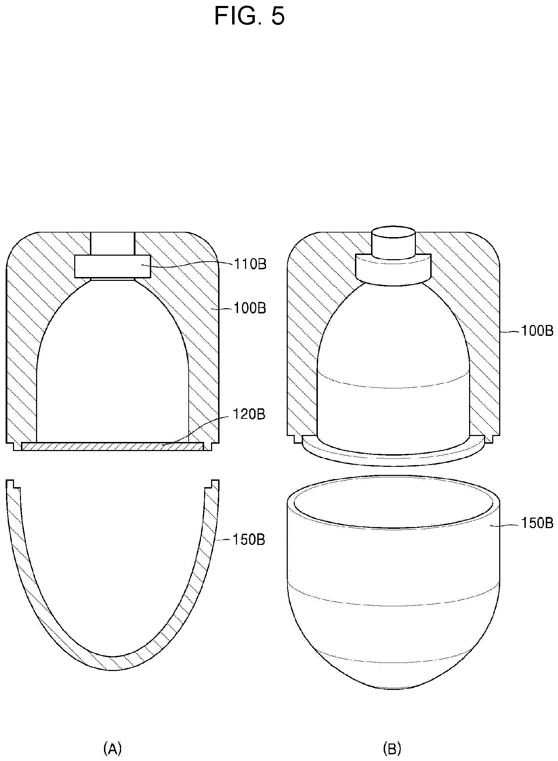

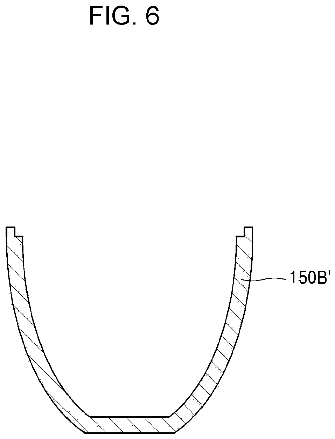

FIG. 4 is a view of a structure of an extracorporeal shock wave generator according to another embodiment of the present invention. FIG. 5 is an exploded view of the extracorporeal shock wave generator shown in FIG. 4. FIG. 6 is a view showing another embodiment of a shock wave transmission unit shown in FIG. 4.

Referring to FIGS. 4 and 5, compared to the extracorporeal shock wave generator 10A of FIG. 1, an extracorporeal shock wave generator 10B according to another embodiment of the present invention does not include the cover 130A and the coupling unit 140A, and includes a shock wave transmission unit 150B of a different type. Structures and functions of a housing 100B and a window 110B of FIG. 5 may be substantially the same as those of the housing 100A and the window 110A of FIG. 1.

The shock wave transmission unit 150B of FIG. 5 may be formed in a convex cap shape in a direction in which the shock wave SW is transmitted (e.g., downward).

The housing 100B and the shock wave transmission unit 150B are directly coupled, and a buffer unit 120B may be provided between the housing 100B and the shock wave transmission unit 150B.

An open area of the housing 100B is sealed by the buffer unit 120B, and a first space SP1 sealed by the housing 100B and the buffer unit 120B may be filled with a shock wave generating medium.

An open area of the shock wave transmission unit 150B may be sealed by the buffer unit 120B, and a second space SP2 sealed by the buffer unit 120B and the shock wave transmission unit 150B may be filled with a shock wave transmission medium.

The first space SP1 and the second space SP2 may be completely separated from each other by the buffer unit 120B.

An intensity of the shock wave SW generated by the reaction RCT between the laser beam LB and the shock wave generating medium may be controlled by the buffer unit 120B, and the shock wave SW transmitted through the buffer unit 120B may be transmitted to the object OBJECT through the shock wave transmission unit 150B and the shock wave transmission medium filled in the second space SP2.

According to an embodiment, the shock wave transmission medium may be composed of water, but is not limited thereto.

Because the shock wave transmission medium does not need to react with the laser beam LB, the shock wave transmission medium may be composed of a material different from that of the shock wave generating medium.

Referring together with FIG. 6, according to an embodiment, a shock wave transmission unit 150B' may be formed in a convex cap shape in a direction in which a shock wave is transmitted, and an end thereof may be formed in a flat surface shape.

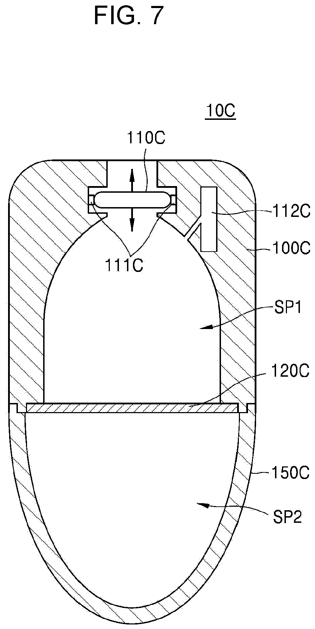

FIG. 7 is a view of a structure of an extracorporeal shock wave generator according to another embodiment of the present invention.

Referring to FIGS. 4 and 7, compared to the extracorporeal shock wave generator 10B of FIG. 4, an extracorporeal shock wave generator 10C has a different shape of a window 110C and a structural difference in that the extracorporeal shock wave generator 10C further includes an coupling member 111C and a shock wave generating medium adjustor 112C.

According to an embodiment, the window 110C is composed of a lens capable of condensing the laser beam LB, for example, a convex lens, and may be movable in height according to a target transmission depth of a set shock wave.

According to an embodiment, the coupling member 111C for coupling a housing 100C and the window 110C may be around the window 110C.

According to an embodiment, the height of the window 110C may be moved by a user applying physical force through a switch (not shown) external to the extracorporeal shock wave generator 10C.

According to another embodiment, the extracorporeal shock wave generator 10C may further include a drive system such as a motor for moving the height of the window 110C electrically.

The extracorporeal shock wave generator 10C may include the shock wave generating medium adjustor 112C for adjusing the amount of shock wave generating medium in the first space SP1 of the housing 100C according to the height movement of the window 110C.

At least a part of the space of the shock wave generating medium adjustor 112C may include the same material as that of the shock wave generating medium.

According to an embodiment, the height of the top of the shock wave generating medium filled in the shock wave generating medium adjustor 112C is higher than the height of the top of the first space SP1 of the housing 100C, and the shock wave generating medium adjustor 112C may have an empty space therein.

According to an embodiment, because the height of the window 110C increases, the shock wave generating medium contained in the shock wave generating medium adjustor 112C may move to the first space SP1 of the housing 100C and fill the first space SP1.

According to an embodiment, because the height of the window 110C decreases, the shock wave generating medium contained in the first space SP1 of the housing 100C may move to the shock wave generating medium adjustor 112C.

Hereinabove, the present invention has been described with reference to the preferred embodiments. However, it will be appreciated by one of ordinary skill in the art that various modifications and changes of the present invention can be made without departing from the scope of the inventive concept which are defined in the appended claims and their equivalents.

## Claims

1. An extracorporeal shock wave generator comprising:
a housing in which an inner space is filled with a shock wave generating medium;
a window coupled to the housing to transmit a laser beam from an outside of the housing to an inside of the housing; and
a shock wave transmission unit coupled to the housing and configured to transmit a shock wave generated as the laser beam reacts to the shock wave generating medium inside the housing to the outside.

2. The extracorporeal shock wave generator of claim 1, further comprising:
a coupling unit configured to couple the housing and the shock wave transmission unit.

3. The extracorporeal shock wave generator of claim 1, further comprising:
a buffer unit configured to reduce a intensity of the generated shock wave.

4. The extracorporeal shock wave generator of claim 3, wherein the buffer unit has elasticity and is formed in a plate shape.

5. The extracorporeal shock wave generator of claim 4, wherein the buffer unit is placed in close contact with a cover covering an opening of the housing or one surface of the shock wave transmission unit.

6. The extracorporeal shock wave generator of claim 1, wherein the shock wave transmission unit is an acoustic lens having one surface flat and the other surface concave.

7. The extracorporeal shock wave generator of claim 6, wherein, when the extracorporeal shock wave generator transmits a shock wave to an object on which a gel-state transmission medium is applied, as the transmission medium flows into a concave portion of the shock wave transmission unit, the shock wave transmission unit comes into close contact with the object.

8. The extracorporeal shock wave generator of claim 1, wherein the shock wave transmission unit is an acoustic lens having one surface flat and the other surface convex.

9. The extracorporeal shock wave generator of claim 1, further comprising:
a buffer unit configured to seal a first space filled with the shock wave generating medium in the housing, and
a second space sealed by the buffer unit and the shock wave transmission unit is filled with a shock wave transmission medium.

10. The extracorporeal shock wave generator of claim 9, wherein the shock wave transmission unit is formed in a convex cap shape in a direction in which a shock wave is transmitted.

11. The extracorporeal shock wave generator of claim 10, wherein the shock wave transmission unit is formed in a convex cap shape in a direction in which a shock wave is transmitted, and an end thereof is formed in a flat surface shape.

12. The extracorporeal shock wave generator of claim 9, wherein the window is composed of a lens capable of condensing the laser beam, and
is movable in height according to a set target shock wave transmission depth.

13. The extracorporeal shock wave generator of claim 12, further comprising:
a shock wave generating medium adjustor configured to control an amount of shock wave generating medium in the first space according to height movement of the window.

14. The extracorporeal shock wave generator of claim 1, wherein a top portion of the inner space of the housing has a dome-shaped cross section.

15. The extracorporeal shock wave generator of claim 1, wherein the shock wave transmission unit is formed of a low-density plastic material.
